# FASCICULE DE BREVET EUROPEEN

(11) **EP 2 181 702 B1**
(45) Date de publication et mention de la délivrance du brevet: **19.07.2017**
(21) Numéro de dépôt: 09174250.2
(22) Date de dépôt: 27.10.2009
(51) Int. Cl.: A61K 8/99, A61Q 5/00

(54) **Utilisation cosmétique d'un lysat de Bifidobacterium species pour le traitement du cuir chevelu gras**
Verwendung eines Bifidobacterium-Lysats zur Behandlung von fettigem Haar
Use of a bifidobacterium lysate for treating greasy hair

(30) Priorité: 28.10.2008 FR 0857333; 05.11.2008 US 111426
(43) Date de publication de la demande: 05.05.2010
(73) Titulaire: L'Oréal, 75008 Paris (FR)
(72) Inventeur: Gueniche, Audrey, 92500 Rueil Malmaison (FR); Bernard, Dominique, 92170 Vanves (FR); Castiel, Isabelle, 06200 Nice (FR)
(74) Mandataire: Nony

(56) Documents cités:
- EP-A- 1 110 555
- WO-A-02/28402
- FR-A- 2 912 917
- US-A1- 2006 008 453

## Description

La présente invention vise principalement à proposer un nouvel actif pour la prévention et/ou le traitement des désordres, notamment esthétiques, associés au cuir chevelu gras.

Le cuir chevelu est un épiderme à renouvellement continuel, comme le reste du tissu cutané, et riche en glandes sébacées.

La sécrétion de sébum est un phénomène normal et utile à la peau comme à la chevelure. Le sébum protège le cuir chevelu et assure la brillance des cheveux en lubrifiant la cuticule.

Malheureusement, une hypersécrétion de sébum, ou séborrhée, peut entraîner des désagréments, des sensations d'inconfort, des désordres esthétiques, voire une pathologie cutanée. Ainsi, une sécrétion excessive de sébum peut se traduire par une peau grasse, voire acnéique, et favoriser l'apparition d'un état pelliculaire gras du cuir chevelu ou pellicules grasses.

Les états pelliculaires gras sont des états chroniques, fréquents, récidivants, et socialement invalidants du fait de leur caractère inesthétique manifeste. De nombreux facteurs peuvent amplifier ces phénomènes et conduire alors à l'apparition de troubles supplémentaires tels que des états inflammatoires du cuir chevelu. Ces états pelliculaires gras et/ou états inflammatoires du cuir chevelu se traduisent par une altération de la fonction barrière de l'épiderme. Qui plus est, ces états peuvent générer des sensations de démangeaison ou prurit, conduisant à des comportements de grattage amplifiant le phénomène d'apparition des pellicules.

Par exemple, le stress, la période hivernale ou la colonisation de la peau ou des follicules pileux par la levure *Malassezia sp.* sont des facteurs renforçant ces états chez la majorité des sujets.

La levure *Malassezia sp.* représente environ 45 % de la flore commensale normale à la surface du cuir chevelu chez des sujets sans pellicule, mais peut représenter 75 % de la flore en cas de pellicules, et jusqu'à 85 % en cas de dermite séborrhéique associée. Les autres germes présents à la surface du cuir chevelu sont des microcoques et des *Propionobacterium.*

Or, il a été récemment démontré que les levures du type *Malassezia* possèdent une activité lipase importante conduisant à l'hydrolyse des triglycérides du sébum en acides gras. Ces acides gras sont alors capables d'entraîner les états pelliculaires chez les personnes sensibles, c'est-à-dire possédant une fonction barrière altérée, et donc plus particulièrement susceptibles à l'action destructrice des acides gras sur la barrière cutanée.

Ainsi, les pellicules grasses se développent d'autant plus facilement que la présence de sébum est importante. Par ailleurs, elles ont tendance à être plus facilement prurigineuses.

Ce désordre esthétique peut être en partie neutralisé *via* différents traitements antifongiques locaux ou systémiques. Ainsi, différentes préparations combinant antifongiques et anti-séborrhéiques ont été proposées afin de traiter les états gras pelliculaires sévères. Les traitements à base d'antifongiques démontrent une certaine efficacité sur les états pelliculaires gras.

Cependant, l'efficacité de ces traitements n'est que suspensive et implique un suivi rigoureux de la part de l'utilisateur (fréquence d'utilisation et temps d'application suffisant).

En conséquence, de nombreux échecs surviennent dans la mise en oeuvre de ces traitements et sont habituellement imputables aux facteurs suivants : mauvais suivi du protocole ; non respect de la fréquence d'utilisation ; aspect non cosmétique du produit, irritation de la base lavante ; mauvaise observance de la durée d'application ; lassitude.

Il demeure donc un besoin de disposer de nouveaux actifs susceptibles d'exercer une action cosmétique bénéfique sur les états du cuir chevelu gras.

Il subsiste également un besoin de disposer d'actifs permettant de rétablir l'écoflore du cuir chevelu et notamment de prévenir la colonisation excessive du cuir chevelu par *Malassezia sp.*

Il existe également un besoin de disposer de nouvelles compositions efficaces pour prévenir et/ou traiter les états du cuir chevelu gras et qui soient agréables et confortables à mettre en oeuvre, favorisant ainsi l'observance du traitement.

La présente invention a pour objet de satisfaire ces besoins.

Ainsi, l'invention concerne l'utilisation cosmétique d'un lysat d'au moins un microorganisme du genre *Bifidobacterium longum* pour traiter et/ou prévenir les états du cuir chevelu gras.

De manière inattendue, les inventeurs ont observé que certains microorganismes probiotiques, de type Bifidobacteria, peuvent s'avérer particulièrement efficaces pour améliorer et restaurer les états du cuir chevelu gras.

Ainsi, comme il ressort des données présentées dans les exemples, les inventeurs ont notamment caractérisé l'aptitude de certains de ces microorganismes à stimuler la synthèse d'un nombre surprenant de protéines susceptibles de favoriser et renforcer les défenses antimicrobiennes de l'épiderme. Les inventeurs ont démontré qu'un lysat de *Bifidobacterium longum* permettait de stimuler la synthèse des protéines telles que la Ribonucléase 7 (Q9H1E1), la dermcidine (P81605), la prolactin - inducible protein (P12273), les protéines S100 A8 et A9 (P05109 et P06702), et la protéine histone (Q5R2W0), aptes à renforcer les défenses de l'épiderme contre la colonisation excessive de microorganismes pathogènes.

Ainsi, la mise en oeuvre des compositions cosmétiques ou dermatologiques ou pharmaceutiques de l'invention favorise la diminution de la colonisation du cuir chevelu et des follicules pileux par *Malassezia sp.*

Les compositions de l'invention peuvent par conséquent avantageusement permettre de rétablir une écoflore équilibrée *via* l'induction de protéines de défenses épidermiques.

Selon un autre de ses aspects, l'invention a pour objet un procédé notamment cosmétique pour traiter et/ou prévenir les désordres esthétiques du cuir chevelu gras chez un sujet comprenant au moins une étape d'administration audit sujet, d'un lysat d'au moins un microorganisme du genre *Bifidobacterium longum*. Selon une variante de réalisation de l'invention, un lysat selon l'invention peut être mis en oeuvre par voie orale.

Selon une autre variante de réalisation de l'invention, le lysat selon l'invention peut être mis en oeuvre par voie topique.

Comme précisé ci-après, les compositions le contenant sont formulées pour être compatibles avec le mode d'administration retenu.

L'invention concerne la mise en oeuvre d'un lysat conforme à l'invention sous forme de composition cosmétique ou dermatologique ou pharmaceutique.

### Microorganismes

Comme précisé précédemment, les microorganismes du genre *Bifidobacterium longum* utilisés à titre d'actifs selon l'invention sont mis en oeuvre sous la forme d'un lysat.

Un lysat désigne communément un matériau obtenu à l'issue de la destruction ou dissolution de cellules biologiques par un phénomène dit lyse cellulaire provoquant ainsi la libération des constituants biologiques intracellulaires naturellement contenus dans les cellules du microorganisme considéré.

Au sens de la présente invention, le terme lysat est utilisé indifféremment pour désigner l'intégralité du lysat obtenu par lyse du microorganisme concerné ou seulement une fraction de celui-ci.

Ainsi, l'invention concerne la mise en oeuvre d'un lysat de *Bifidobacterium longum.* Le lysat mis en oeuvre est donc formé en tout ou partie des constituants biologiques intracellulaires et des constituants des parois et membranes cellulaires.

Plus précisément, il contient la fraction cytoplasmique cellulaire renfermant les enzymes tels que la déhydrogénase d'acide lactique, les phosphatases, les phosphokétolases, et transaldolases et les métabolites. A titre illustratif, les constituants des parois cellulaires sont notamment le peptidoglycane, la muréine ou mucopeptide et l'acide teichoique et les constituants des membranes cellulaires sont composés de glycérophospho lipide.

Cette lyse cellulaire peut être accomplie par différentes technologies, telles que par exemple un choc osmotique, un choc thermique, par ultrasons, ou encore sous contrainte mécanique de type centrifugation.

Selon un mode de réalisation préféré, le lysat est obtenu par désintégration aux ultra-sons.

Plus particulièrement, ce lysat peut être obtenu selon la technologie décrite dans le brevet US 4,464,362, et notamment selon le protocole suivant.

Un microorganisme de type *Bifidobacterium species* considéré est cultivé anaérobiquement dans un milieu de culture adéquat, par exemple selon les conditions décrites dans les documents US 4,464,362 et EP 0 043 128. Lorsque la phase stationnaire du développement est atteinte, le milieu de culture peut être inactivé par pasteurisation, par exemple à une température de 60 à 65 °C pendant 30 mn. Les microorganismes sont alors recueillis par une technique de séparation conventionnelle par exemple filtration membranaire, centrifugé et remis en suspension dans une solution stérile de NaCl à une concentration physiologique. Le lysat peut être obtenu par désintégration aux ultra-sons d'un tel milieu afin d'en libérer les fractions cytoplasmiques, les fragments de paroi cellulaire et les produits issus du métabolisme. Puis tous les composants dans leur distribution naturelle sont ensuite stabilisés dans une solution aqueuse faiblement acide.

On obtient ainsi généralement un lysat possédant une concentration de l'ordre de 0,1 à 50 %, en particulier de 1 à 20 % et notamment environ 5 % en poids de matière(s) active(s) par rapport à son poids total.

Le lysat peut être mis en oeuvre sous différentes formes, sous la forme d'une solution ou sous une forme pulvérulente.

Il peut avantageusement s'agir du lysat enregistré sous le nom INCI : Bifidat ferment Lysate, sous le nom EINECS: Bifidobacterium longum, sous le N° EINECS: N° 306-168-4 et sous le N° CAS : N° 96507-89-0.

Le produit commercialisé sous la dénomination Repair Complex CLR^{®} par la société K. RICHTER GmbH et qui est formé d'un lysat inactivé de l'espèce *Bifidobacterium longum,* entre dans le cadre de l'invention.

L'actif formant le lysat et appartenant à l'espèce *Bifidobacterium longum* peut être formulé dans une composition à raison d'au moins 0,0001 % (exprimé en poids sec), en particulier à raison de 0,001 à 20 % et plus particulièrement à raison de 0,01 à 2 % en poids sec de matière active par rapport au poids total du support ou de la composition le contenant.

Dans le cas particulier où le(s) microorganisme(s) est (sont) formulé(s) dans des compositions à administrer par voie orale, la concentration en microorganisme(s) peut être ajustée de manière à correspondre à des doses (exprimées en équivalent de microorganisme) variant de 5.10² à 10¹³ ufc/j et en particulier de 10⁵ à 10¹¹ ufc/j. va de soi qu'un lysat de l'invention est mis en oeuvre en quantité efficace.

Par « quantité efficace », on entend au sens de la présente invention une quantité suffisante pour obtenir l'effet attendu.

D'une manière générale, une composition à application topique selon l'invention comprend généralement de 0,0001 à 30 %, en particulier de 0,001 à 15 % et plus particulièrement de 0,1 à 10 % en un ou plusieurs microorganismes notamment probiotiques, annexes.

Ce ou ces microorganisme(s) peu(ven)t être inclus dans une composition selon l'invention sous une forme vivante, semi-active ou inactivée, morte.

Il(s) peu(ven)t également être inclus sous forme de fractions de composants cellulaires ou sous la forme de métabolites. Le ou le(s) microorganisme(s), métabolite(s) ou fraction(s) peu(ven)t également être introduit(s) sous la forme d'une poudre, d'un liquide, d'un surnageant de culture ou l'une de ses fractions, dilué(e) ou non, ou encore concentré(e) ou non.

Dans le cas où les microorganismes sont formulés dans une composition sous une forme vivante, la quantité de microorganismes vivants peut varier de 10³ à 10¹⁵ ufc/g, en particulier de 10⁵ à 10¹⁵ ufc/g et plus particulièrement de 10⁷ à 10¹² ufc/g de microorganismes par gramme de composition.

Les compositions selon l'invention peuvent se présenter sous toutes les formes galéniques normalement disponibles pour le mode d'administration retenu.

Le support peut être de nature diverse selon le type de composition considérée.

En ce qui concerne plus particulièrement les compositions destinées à une administration par voie topique, il peut s'agir d'une solution aqueuse, hydroalcoolique ou huileuse, d'une dispersion du type solution ou dispersion du type lotion ou sérum, d'une émulsion de consistance liquide ou semi-liquide du type lait, obtenue par dispersion d'une phase grasse dans une phase aqueuse (H/E) ou inversement (E/H), ou d'une suspension ou émulsion de consistance molle, semi-solide ou solide, du type crème, de gel aqueux ou anhydre, ou encore de microémulsions, de microcapsules, de microparticules, ou de dispersions vésiculaires de type ionique et/ou non ionique.

Ces compositions sont préparées selon les méthodes usuelles.

Ces compositions peuvent notamment constituer des crèmes de nettoyage, de protection, de traitement ou de soin des lotions, gels ou mousses pour le soin du cuir chevelu, comme des lotions de nettoyage ou de désinfection ou des compositions pour le bain.

Les compositions selon l'invention peuvent également consister en des préparations solides constituant des savons ou des pains de nettoyage.

Elles peuvent être également utilisées pour le cuir chevelu sous forme de solutions, de crèmes, de gels, d'émulsions, de mousses ou encore sous forme de compositions pour aérosol contenant également un agent propulseur sous pression.

Lorsque la composition de l'invention est une émulsion, la proportion de la phase grasse peut aller de 5 à 80 % en poids, et de préférence de 10 à 50 % en poids par rapport au poids total de la composition. Les huiles, les émulsionnants et les co-émulsionnants utilisés dans la composition sous forme d'émulsion sont choisis parmi ceux classiquement utilisés dans le domaine cosmétique et/ou dermatologique. L'émulsionnant et le co-émulsionnant peuvent être présents, dans la composition, en une proportion allant de 0,3 à 30 % en poids, et de préférence de 0,5 à 20 % en poids par rapport au poids total de la composition.

Lorsque la composition de l'invention est une solution ou un gel huileux, la phase grasse peut représenter plus de 90 % du poids total de la composition.

De façon connue, les formes galéniques dédiées à une administration topique peuvent contenir également des adjuvants habituels dans le domaine cosmétique, pharmaceutique et/ou dermatologique, tels que les gélifiants hydrophiles ou lipophiles, les actifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse et/ou dans la phase aqueuse.

Comme matières grasses utilisables dans l'invention, on peut citer les huiles minérales comme par exemple le polyisobutène hydrogéné et l'huile de vaseline, les huiles végétales comme par exemple une fraction liquide du beurre de karité, huile de tournesol et d'amandes d'abricot, les huiles animales comme par exemple le perhydrosqualène, les huiles de synthèse notamment l'huile de Purcellin, le myristate d'isopropyle et le palmitate d'éthyl hexyle, les acides gras insaturés et les huiles fluorées comme par exemple les perfluoropolyéthers. On peut aussi utiliser des alcools gras, des acides gras comme par exemple l'acide stéarique et comme par exemple des cires notamment de paraffine, carnauba et la cire d'abeilles. On peut aussi utiliser des composés siliconés comme les huiles siliconées et par exemple les cyclométhicone et diméthicone, les cires, les résines et les gommes siliconées.

Comme émulsionnants utilisables dans l'invention, on peut citer par exemple le stéarate de glycérol, le polysorbate 60, le mélange alcool cétylstéarylique/alcool cétylstéarylique oxyéthyléné à 33 moles d'oxyde d'éthylène vendu sous la dénomination Sinnowax AO^{®} par la société HENKEL, le mélange de PEG-6/PEG-32/Glycol Stéarate vendu sous la dénomination de Tefose^{®} 63 par société GATTEFOSSE, le PPG-3 myristyl éther, les émulsionnants siliconés tels que le cétyldiméthicone copolyol et le mono- ou tristéarate de sorbitane, le stéarate de PEG-40, le monostéarate de sorbitane oxyéthyléné (200E).

Comme solvants utilisables dans l'invention, on peut citer les alcools inférieurs, notamment l'éthanol et l'isopropanol, le propylène glycol.

Une composition de l'invention peut également contenir de façon avantageuse une eau thermale et/ou minérale, notamment choisie parmi l'eau de Vittel, les eaux du bassin de Vichy et l'eau de la Roche Posay.

Comme gélifiants hydrophiles, on peut citer les polymères carboxyliques tel que le carbomer, les copolymères acryliques tels que les copolymères d'acrylates/alkylacrylates, les polyacrylamides et notamment le mélange de polyacrylamide, C13-14-Isoparaffine et Laureth-7 vendu sous le nom de Sepigel 305^{®} par la société SEPPIC, les polysaccharides comme les dérivés cellulosiques tels que les hydroxyalkylcelluloses et en particulier les hydroxypropylcellulose et hydroxyéthylcellulose, les gommes naturelles telles que les guar, caroube et xanthane et les argiles.

Comme gélifiants lipophiles, on peut citer les argiles modifiées comme les bentones, les sels métalliques d'acides gras comme les stéarates d'aluminium et la silice hydrophobe, ou encore l'éthylcellulose et le polyéthylène.

Dans le cas d'une utilisation d'une association conforme à l'invention par voie orale, on privilégie l'utilisation d'un support ingérable.

Le support ingérable peut être de diverses natures selon le type de composition considéré.

Conviennent notamment comme supports alimentaires ou pharmaceutiques, le lait, le yaourt, le fromage, les laits fermentés, les produits fermentés à base de lait, des glaces, des produits à base de céréales fermentées, des poudres à base de lait, des formules pour enfants et nourrissons, des produits alimentaires de type confiserie, chocolat, céréales, des aliments pour animaux en particulier domestiques, des comprimés, gélules ou tablettes, des suppléments oraux sous forme sèche et les suppléments oraux sous forme liquide.

Un microorganisme de l'invention, et/ou l'une de ses fractions, peut être par ailleurs formulé avec les excipients et composants usuels pour de telles compositions orales ou compléments alimentaires, à savoir notamment composants gras et/ou aqueux, agents humectants, épaississants, conservateurs, agents de texture, de saveur et/ou d'enrobage, antioxydants, conservateurs et colorants usuels dans le domaine de l'alimentaire.

Les agents de formulation et excipients pour composition orale, et notamment pour compléments alimentaires, sont connus dans ce domaine et ne font pas ici l'objet d'une description détaillée. Pour l'ingestion, de nombreuses formes de réalisation de compositions orales et notamment de compléments alimentaires sont possibles. Leur formulation est réalisée par les procédés usuels pour produire des dragées, gélules, gels, hydrogels à libération contrôlée, émulsions, comprimés, capsules.

En particulier, le microorganisme selon l'invention peut être incorporé dans toute autre forme de compléments alimentaires ou d'aliments enrichis, par exemple des barres alimentaires, ou des poudres compactées ou non. Les poudres peuvent être diluées à l'eau, dans du soda, des produits laitiers ou dérivés du soja, ou être incorporées dans des barres alimentaires.

Selon un mode de réalisation particulier, les microorganismes annexes considérés selon l'invention peuvent être formulés au sein de compositions sous une forme encapsulée de manière à améliorer significativement leur durée de survie. Dans un tel cas, la présence d'une capsule peut en particulier retarder ou éviter la dégradation du microorganisme au niveau du tractus gastro intestinal.

Quel que soit le mode d'administration considéré, un lysat de l'invention peut avantageusement être associé à au moins un autre actif.

Ainsi, une composition topique ou orale, ou une association selon l'invention peuvent en outre contenir au moins un actif anti-séborrhéique et/ou au moins un actif antipelliculaire.

Une telle formulation permet avantageusement d'amplifier les effets bénéfiques d'un lysat de l'invention.

On entend par actif anti-séborrhéique un composé capable de réguler l'activité des glandes sébacées.

Un actif anti-séborrhéique convenant à l'invention peut, notamment, être choisi parmi l'acide rétinoïque, le peroxyde de benzoyle, le soufre, la vitamine B6 (ou pyridoxine), le chlorure de sélénium, la criste marine ; les mélanges d'extrait de canelle, de thé et d'octanoylglycine tel que le Sepicontrol A5 TEA^{®} de chez Seppic ; le mélange de canelle, de sarcosine et d'octanoylglycine, commercialisé notamment par la société SEPPIC sous la dénomination commerciale Sepicontrol A5^{®} ; les sels de zinc tels que le gluconate de zinc, le pyrrolidone carboxylate de zinc (ou pidolate de zinc), le lactate de zinc, l'aspartate de zinc, le carboxylate de zinc, le salicylate de zinc, le cystéate de zinc ; les dérivés de cuivre et en particulier le pidolate de cuivre tel que Cuivridone^{®} de Solabia ; des extraits de végétaux des espèces *Arnica montana*, *Cinchona succirubra*, *Eugenia caryophyllata*, *Humulus lupulus, Hypericum perforatum*, *Mentha piperita*, *Rosmarinus officinalis, Salvia oficinalis* et *Thymus vulgaris,* tous commercialisés par exemple par la société MARUZEN ; les extraits de reine des prés *(spiraea ulamaria)* tel que celui vendu sous la dénomination Sébonormine^{®} par la société Silab ; les extraits d'algue *Laminaria saccharina* tel que celui vendu sous la dénomination Phlorogine^{®} par la société Biotechmarine ; les mélanges d'extraits de racines de pimprenelle (*Sanguisorba officinalis*/*Poterium officinale*), de rhizomes de gingembre (*Zingiber officinalis)* et d'écorce de cannelier *(Cinnamomum cassia*) tel que celui vendu sous la dénomination Sebustop^{®} par la société Solabia ; les extraits de graines de lin tel que celui vendu sous la dénomination Linumine^{®} par la société Lucas Meyer ; les extraits de Phellodendron tels que ceux vendu sous la dénomination Phellodendron extract BG par la société Maruzen ou Oubaku liquid B par la société Ichimaru Pharcos ; les mélanges d'huile d'argan, d'extrait de *Serenoa serrulata* (saw palmetto) et d'extrait de graines de sésame tel que celui vendu sous la dénomination Regu SEB^{®} par la société Pentapharm ; les mélanges d'extraits d'epilobe, de *Terminalia chebula,* de capucine et de zinc biodisponible (microalgues) tel que celui vendu sous la dénomination Seborilys^{®} par la société Green Tech ; les extraits de *Pygeum afrianum* tel que celui vendu sous la dénomination Pygeum afrianum sterolic lipid extract par la société Euromed ; les extraits de *Serenoa serrulata* tels que ceux vendus sous les dénomination Viapure Sabal par la société Actives International, ou ceux vendus par la société Euromed ; les mélanges d'extraits de plantain, de *Berberis aquifolium* et de salicylate de sodium tels que celui vendu sous la dénomination Seboclear^{®} par la société Rahn ; l'extrait de clou de girofle tel que celui vendu sous la dénomination Clove extract Powder par la société Maruzen ; l'huile d'argan telle que celle vendue sous la dénomination Lipofructyl^{®} par les Laboratoires Sérobiologiques ; les filtrats de protéine lactique tels que celui vendu sous la dénomination Normaseb^{®} par la société Sederma ; les extraits d'algue *Laminaria*, tel que celui vendu sous la dénomination Laminarghane^{®} par la société Biotechmarine ; les oligosaccharides d'algue *Laminaria digitata* tel que celui vendu sous la dénomination Phycosaccharide AC par la société Codif ; les extraits de sucre de canne tel que celui commercialisé sous la dénomination Policosanol^{®} par la société Sabinsa ; l'huile de schiste sulfonée, telle que celle vendue sous la dénomination Ichtyol Pale^{®} par la société Ichthyol ; les extraits d'ulmaire (*spiraea ulmaria)* tels que celui vendu sous la dénomination Cytobiol^{®} Ulmaire par la société Libiol ; l'acide sébacique, notamment vendu sous la forme d'un gel de polyacrylate de sodium sous la dénomination Sebosoft^{®} par la société Sederma ; les glucomannanes extraits de tubercule de konjac et modifié par des chaînes alkylsulfonates tel que celui vendu sous la dénomination Biopol Beta par la société Arch Chemical ; les extraits de *Sophora angustifolia*, tels que ceux vendus sous la dénomination Sophora powder ou Sophora extract par la société Bioland ; les extraits de *Cinchona succirubra* bark tel que celui vendu sous la dénomination le Red bark HS par la société Alban Muller ; les extraits de *Quillaja saponaria* tel que celui vendu sous la dénomination Panama wood HS par la société Alban Muller ; la glycine greffée sur chaîne undécylénique, telle que celle vendue sous la dénomination Lipacide UG OR par la société Seppic ; le mélange d'acide oléanolique et d'acide nordihydroguaïarétique, tel que celui vendus sous la forme d'un gel sous la dénomination AC.Net par la société Sederma ; l'acide phthalimidoperoxyhexanoïque ; le citrate de trialkyle(C₁₂-C₁₃) vendu sous la dénomination COSMACOL^{®} ECI par la société Sasol; le citrate de trialkyle(C₁₄-C₁₅) vendu sous la dénomination COSMACOL^{®} ECL par la société Sasol ; l'acide 10-hydroxydécanoïque, et notamment les mélanges d'acide 10-hydroxydécanoïque, d'acide sébacique et de 1,10-décandiol tels que celui vendu sous la dénomination Acnacidol^{®} BG par la société Vincience ; et leurs mélanges.

L'actif anti-séborrhéique est par exemple présent dans une teneur allant de 0,1 à 10 % en poids, de préférence de 0,1 à 5 % en poids, et préférentiellement de 0,5 % à 3 % en poids, par rapport au poids total de la composition.

On entend par actif anti-pelliculaire un composé capable de prévenir l'apparition des pellicules, diminuer leur nombre et/ou les faire disparaître totalement.

Un actif anti-pelliculaire convenant à l'invention peut, notamment, être choisi parmi :
- les sels de pyridinethione notamment les sels de calcium, de magnésium, de barium, de strontium, de zinc, de cadmium, d'étain et de zirconium. Le sel de zinc de pyridinethione est particulièrement préféré. Le sel de zinc de pyridinethione est notamment commercialisé sous la dénomination Omadine de zinc par la société OLIN.
- les trihalogéno carbamide de formule: 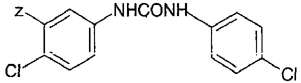 dans laquelle Z représente un atome d'halogène comme le chlore ou un groupement trihalogénoalkyle en C₁-C₄ tel que CF₃.
- le triclosan représenté par la formule : 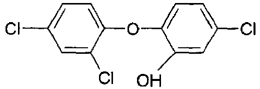
- les composés azolés tels que le climbazole, le kétoconazole, le clotrinazole, l'econazole, l'isoconazole et le miconazole.
- les polymères antifongiques tels que l'amphotéricine B ou la nystatine.
- les sulfures de sélénium en particulier ceux de formule Sₓ Se ₈₋ₓ, x allant de 1 à 7.
- le soufre sous ses différentes formes, le sulfure de cadmium, l'allantoïne, les goudrons de houille ou de bois et leurs dérivés en particulier l'huile de cade, l'acide salicylique, l'acide undécylénique, l'acide fumarique, les allylamines telle que la terbinafine.

A titre d'exemples préférentiels d'agents antipelliculaires, on peut notamment citer la pyrithione de zinc, l'acide salicylique, le disulfure de sélénium, et leurs mélanges.

Une composition selon l'invention comprend avantageusement de 0,001 à 10 % en poids d'agent(s) antipelliculaire(s), de préférence de 0,1 à 5 % en poids, encore plus préférentiellement de 0,2 à 2 % en poids, par rapport au poids total de la composition.

Les compositions topiques ou orales, ou associations selon l'invention peuvent également contenir à titre d'un actif additionnel, un actif notamment destiné à renforcer la barrière cutanée tel qu'un actif favorisant par exemple la maturation du stratum corneum, par exemple un actif hydratant, un agent prodesquamant, ou un actif favorisant la différentiation tardive de l'épiderme et les activités enzymatiques qui y sont liées.

A titre d'actifs conventionnellement mis en oeuvre, on peut citer, les vitamines B3, B5, B6, B8, C, E, ou PP, la niacine, les caroténoïdes, les polyphénols et minéraux tels que zinc, calcium, magnésium. Par "agent prodesquamant », on entend tout composé capable d'agir :
- soit directement sur la desquamation en favorisant l'exfoliation, tel que les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique ; l'urée et certains de ses dérivés; l'acide gentisique ; les oligofucoses ; l'acide cinnamique ; l'acide dioïque ; l'extrait de Saphora japonica ; le resvératrol ; les détergents et certains dérivés d'acide jasmonique ;
- et/ou sur les activités des enzymes impliquées dans la dégradation des cornéodesmosomes, telles que la stratum corneum chymotryptic enzyme (SCCE) voire d'autres protéases (trypsin-like, chymotrypsin-like, cathepsin D) ainsi que d'autres catégories d'hydrolases (ex : glycosidases, céramidases). On peut citer les agents chélatant des sels minéraux : l'EDTA ; l'acide N-acyl-N,N',N' éthylène diaminetriacétique ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP-0 852 949, ainsi que le méthyl glycine diacétate de sodium commercialisé par BASF sous la dénomination commerciale TRILON M) ; le miel ; les dérivés de sucre tels que l'O-octanoyl-6-D-maltose et la N-acétyl glucosamine ; l'urée ou certains de ses dérivés par exemple Hydrovance ; les dérivés de C-Glycosides.

En particulier, on peut utiliser, à titre d'actif, un complexe anti-oxydant comprenant les vitamines C et E, et au moins un caroténoïde, notamment un caroténoïde choisi parmi le β-carotène, le lycopène, l'astaxanthine, la zéaxanthine et la lutéine, des flavonoïdes telles que les catéchines, l'hespéridine, des proanthocyanidines et des anthocyanines, des ubiquinones, des extraites de café contenant des polyphénols et/ou des diterpènes, des extraits de chicorés, des extraits de ginkgo biloba, des extraits de raisins riches en proanthocyanidines, des extraits de piment, des extraits de soja, d'autres sources de flavonoïdes possédant des propriétés antioxydantes, des acides gras, des prébiotiques, la taurine, du resveratrol, des acides aminés du sélénium, des précurseurs de glutathion.

Parmi les flavonoïdes, on choisit de préférence les catéchines et les OPC (oligomères procyanidoliques).

Il peut également s'agir d'au moins un prébiotique ou un mélange de prébiotiques. Plus particulièrement, ces prébiotiques peuvent être choisis parmi les oligosaccharides, produits à partir du glucose, galactose, xylose, maltose, sucrose, lactose, amidon, xylane, l'hémicellulose, l'inuline, des gommes de type acacia par exemple, ou un de leurs mélanges. Plus particulièrement, l'oligosaccharide comprend au moins un fructo-oligosaccharide. Plus particulièrement, ce prébiotique peut comprendre un mélange de fructo-oligosaccharide et d'inuline.

Dans les formes galéniques topiques, on peut utiliser plus particulièrement comme actifs hydrophiles les protéines ou les hydrolysats de protéine, les acides aminés, les polyols notamment en C₂ à C₁₀ comme les glycérine, sorbitol, butylène glycol et polyéthylène glycol, l'urée, l'allantoïne, les sucres et les dérivés de sucre, les vitamines hydrosolubles, l'amidon, des extraits bactériens ou végétaux comme ceux d'Aloe Vera.

Pour ce qui est des actifs lipophiles, on peut utiliser le rétinol (vitamine A) et ses dérivés, le tocophérol (vitamine E) et ses dérivés, les céramides, les huiles essentielles et les insaponifiables (tocotriènol, sésamine, gamma oryzanol, phytostérols, squalènes, cires, terpènes).

Comme actifs susceptibles d'être plus particulièrement associés au lysat dans une formule galénique orale, on peut également considérer tous les ingrédients communément utilisés et/ou autorisés.

A titre illustratif, on peut citer les vitamines, les minéraux, les lipides essentiels, les oligoéléments, les polyphénols, les flavonoïdes, les phytoestrogènes, les antioxydants tels que l'acide lipoïque et le coenzyme Q10, les caroténoïdes, les prébiotiques, les protéines et les acides aminés, les mono et polysaccharides, les amino-sucres, les phytostérols et alcools triterpéniques d'origine végétale.

Il s'agit, en particulier, des vitamines A, C, D, E, PP et du groupe B. Parmi les caroténoïdes, on choisit de préférence, le béta-carotène, le lycopène, la lutéine, la zéazanthine et l'astaxanthine. Les minéraux et oligo-éléments particulièrement mis en oeuvre sont le zinc, le calcium, le magnésium, le cuivre, le fer, l'iode, le manganèse, le sélénium, le chrome (III). Parmi les polyphénols, on retient aussi en particulier les polyphénols de raisin, de thé, d'olive, de cacao, de café, de pomme, de myrtille, de sureau, de fraise, de canneberge, et d'oignon. De préférence parmi les phytoestrogènes, on retient les isoflavones sous la forme libre ou glycosylée, telles que la génistéine, la daidzéine, la glycitéine ou encore les lignanes, en particulier ceux du lin et du schizandra chinensis. Les acides aminés ou les peptides et les protéines les contenant, tels que la taurine, la thréonine, la cystéine, le tryptophane, la méthionine. Les lipides appartiennent de préférence au groupe des huiles contenant des acides gras mono et polyinsaturés tels que les acides oléique, linoléique, alpha-linolénique, gamma-linolénique, stearidonique, les acides gras oméga-3 de poisson à longue chaîne tels que l'EPA et le DHA, les acides gras conjugués issus de végétaux ou d'animaux tels que les CLA (Conjugated Linoleic Acid).

Un procédé de traitement cosmétique de l'invention peut être mis en oeuvre notamment en administrant les compositions cosmétiques et/ou dermatologiques ou associations telles que définies ci-dessus, selon la technique d'utilisation habituelle de ces compositions. Par exemple : applications de crèmes, de gels, de sérums, de lotions, sur le cuir chevelu, application d'une lotion sur cheveux mouillés ou de shampooings pour ce qui est de l'application topique.

Un procédé cosmétique selon l'invention peut être ainsi mis en oeuvre par administration topique ou orale, journalière par exemple, du lysat considéré selon l'invention.

Un procédé selon l'invention peut comprendre une administration unique. Selon un autre mode de réalisation, l'administration est répétée par exemple 2 à 3 fois quotidiennement sur une journée ou plus et généralement sur une durée prolongée d'au moins 4 semaines, voire 4 à 15 semaines, avec le cas échéant une ou plusieurs périodes d'interruption.

Dans la description et dans les exemples suivants, sauf indication contraire, les pourcentages sont des pourcentages en poids et les plages de valeurs libellées sous la forme « entre ... et ... » incluent les bornes inférieure et supérieure précisées.

Les ingrédients sont mélangés, avant leur mise en forme, dans l'ordre et dans des conditions facilement déterminées par l'homme de l'art.

La teneur et la nature des ingrédients mis en oeuvre dans les compositions de l'invention sont ajustées par l'homme de l'art de sorte à ne pas affecter substantiellement les propriétés requises pour les compositions de l'invention.

Les exemples ci-après sont présentés à titre illustratif et non limitatif du domaine de l'invention.

### EXEMPLES

### Exemple 1 :

| Lotion pour le cuir chevelu | % en poids |
|---|---|
| Poudre de lysat de *Bifidobacterium longum** | 5,00** |
| Antioxydant | 0,05 |
| Isopropanol | 40,0 |
| Conservateur | 0,30 |
| Eau | qsp 100 |

| | |
|---|---|
| * Il s'agit du lysat enregistré sous le nom INCI Bifidat ferment Lysate, et mis en oeuvre sous la forme d'une formulation à 5 % en poids d'actif ** quantité exprimée en produit total | |

### Exemple 2 :

| Lait pour le soin du cuir chevelu | % en poids |
|---|---|
| Poudre de lysat de *Bifidobacterium longum** | 5,00** |
| Stéarate de glycérol | 1,00 |
| Huile d'alcool cétylstéarylique/alcool cétylstéarylique oxyéthyléné à 30 moles OE (Sinnowax AO^{®} vendu par la société HENKEL) | 3,00 |
| Alcool cétylique | 1,00 |
| Diméthicone (DC 200 Fluid^{®} vendu par la société DOW CORNING) | 1,00 |
| Huile de vaseline | 6,00 |
| Myristate d'isopropyle (Estol IMP 1514 vendu^{®} par UNICHEMA) | 3,00 |
| Antioxydant | 0,05 |
| Glycérine | 20,00 |
| Conservateur | 0,30 |
| Eau | qsp 100 |

| | |
|---|---|
| * Il s'agit du lysat enregistré sous le nom INCI Bifidat ferment Lysate, et mis en oeuvre sous la forme d'une formulation à 5 % en poids d'actif ** quantité exprimée en produit total | |

### Exemple 3

| Gel pour le soin du cuir chevelu | % en poids |
|---|---|
| Poudre de lysat de *Bifidobacterium longum** | 5,00** |
| Hydroxypropylcellulose (Klucel H^{®} vendu par la société HERCULES) | 5,00 |
| | 1,00 |
| Vitamine E | 2,50 |
| Antioxydant | 0,05 |
| Isopropanol | 40,00 |
| Conservateur | 0,30 |
| Eau | qsp 100 |

| | |
|---|---|
| * Il s'agit du lysat enregistré sous le nom INCI Bifidat ferment Lysate, et mis en oeuvre sous la forme d'une formulation à 5 % en poids d'actif ** quantité exprimée en produit total | |

### Exemple 4

| Lait pour le soin du cuirs chevelu | % en poids |
|---|---|
| Poudre de lysat de *Bifidobacterium longum** | 5,00** |
| Stéarate de glycérol | 1,00 |
| Alcool cétylstéarylique/alcool cétylstéarylique oxyéthyléné à 3 moles OE (Sinnovax AO^{®} vendu par la société HENKEL) | 3,00 |
| Alcool cétylique | 1,00 |
| Diméthicone (DC 200 Fluid^{®} vendu par la société Dow Corning) | 1,00 |
| Huile de vaseline | 6,00 |
| Myristate d'isopropyle (Estol IPM 1514^{®} vendu par la société Unichema) | 3,00 |
| Glycérine | 20,00 |
| Conservateur | 0,30 |
| Eau | |

| | |
|---|---|
| * Il s'agit du lysat enregistré sous le nom INCI Bifidat ferment Lysate, et mis en oeuvre sous la forme d'une formulation à 5 % en poids d'actif ** quantité exprimée en produit total | |

### Exemple 5

| Crème pour le soin du cuir chevelu | % en poids |
|---|---|
| Arachidyl behenyl alcohol/arachidylglusoside | 3,0 |
| Isohexadecane | 7,0 |
| Poudre de lysat de *Bifidobacterium longum** | 5,00** |
| Glycérine | 2,0 |
| Extrait de *Vitreoscilla filiformis* | 3,0 |
| BHT | 0,05 |
| POB méthyle | 0,1 |
| POB propyle | 0,05 |
| Eau | qsp 100 |

| | |
|---|---|
| * Il s'agit du lysat enregistré sous le nom INCI Bifidat ferment Lysate, et mis en oeuvre sous la forme d'une formulation à 5 % en poids d'actif ** quantité exprimée en produit total | |

### Exemple 6

| Gel pour le soin des cheveux | | % en poids |
|---|---|---|
| Poudre de lysat de *Bifidobacterium longum** | | 5,00** |
| Citrate de Cuivre | | 2,00 |
| Extrait de *Vitreoscilla filiformis* | | 3,00 |
| Antioxydant | | 0,05 |
| Vitamine C | | 2,50 |
| Antioxydant | | 0,05 |
| Isopropanol | | 40,00 |
| Conservateur | | 0,30 |
| Eau | qsp | 100,00 |

| | | |
|---|---|---|
| * Il s'agit du lysat enregistré sous le nom INCI Bifidat ferment Lysate, et mis en oeuvre sous la forme d'une formulation à 5 % en poids d'actif ** quantité exprimée en produit total | | |

### Exemple 7

### Evaluation des effets d'un lysat de Bifidobacterium longum sur un épiderme

Le produit testé est un lysat de *Bifidobacterium longum* en suspension désintégrée (aux ultrasons) dans un milieu aqueux faiblement acide commercialisé sous le nom Repair Complex CLR^{®}.

L'actif a été testé seul dans une étude randomisée en double aveugle.

Soixante six femmes présentant des peaux sèches ont été réparties en deux groupes, placebo (n=33 groupe A), Repair Complex CLR^{®} (n=33 groupe B). Les traitements ont été appliqués topiquement durant 58 jours, l'actif étant formulé à 10 % de la formulation testée. Cette formule support est une émulsion H/E déminéralisée Arlacel/myrj^{®} contenant 5 % Parleam, 15 % de cyclopentasiloxane, 3% glycérine et 2 % vaseline.

Dans la formule placebo, l'absence de lysat est compensée par de l'eau.

Les sujets ont été évalués à J1, J29, J43, et J57.

L'évolution de différents marqueurs cutanés a été étudiée par protéomique différentielle sur des échantillons de *stratum corneum* isolé.

Un prélèvement est effectué au niveau cutané aux temps J1, J29, J43 et J57 par stripping vernis afin de ne prélever qu'une partie du *stratum corneum*, soit au maximum 4 à 5 couches de *stratum corneum*.

Une toile de nylon filtre 41 µm type NY41 Millipore est appliquée sur une zone préalablement définie de la jambe gauche. Puis un vernis transparent de référence 614254/T.D. comprenant : nitrocellulose 6,86 g ; isopropanol 2,94 g ; résine alkyle hypoallergénique 7,35 g ; acetyl tributyl citrate 7,7 g ; acétate d'éthyle 75,15 g ; est étalé à l'aide d'un pinceau (15mm), puis laissée sécher pendant 15 min. La toile de nylon est ensuite récupérée à l'aide d'une pince Brucelles en arrachant d'un coup sec le stripping vernis.

Les strippings vernis sont conservés à -20 °C à plat dans des sachets plastiques.

Ces prélèvements de peau (stripping vernis de stratum corneum) ont ensuite été analysés par protéomique au moyen d'une technique dite de « marquage isobarique », pour évaluer l'expression de différentes protéines.

Cette technique dite de « marquage isobarique » ou iTRAQ repose sur le marquage de peptides tryptiques avec une série de réactifs, dits isobariques car possédant tous une masse moléculaire de 145 Da, et formant un lien covalent avec les amines primaires de l'extrémité amino-terminal ou de la chaîne latérale des résidus lysine.

Les peptides marqués sont détectés par spectrométrie de masse avec la masse intrinsèque du peptide plus 145 Da provenant du réactif. A l'étape de fragmentation du peptide, la contribution de chacun des réactifs est appréciée par la libération d'ions (fragments) ayant des masses différentes spécifiques.

Une telle méthode est plus précisément décrite par Zieske (J. Exp. Bot., 2006, 57 :1501) ou Wiese et al. (Proteomics, 2007,7 :340).

Les résultats de l'analyse par protéomique ont montré que le lysat de *Bifidobactérium longum* stimule l'expression de différentes protéines de défenses antimicrobiennes de l'épiderme telles que la Ribonucléase 7 (n° accession Uniref Q9H1E1), la dermcidine (P81605), la «prolactin-inducible protein» (P12273), les protéines S100 A8 et A9 (P05109 et P06702), et la protéine histone (Q5R2W0).

Un lysat de l'invention permet donc de renforcer la fonction barrière de l'épiderme, et de traiter et/ou prévenir les effets délétères d'une colonisation excessive de l'épiderme par *Malassezia sp.,* notamment vis-à-vis du cuir chevelu gras.

## Revendications

1. Utilisation cosmétique d'un lysat d'au moins un microorganisme de l'espèce *Bifidobacterium longum* pour traiter et/ou prévenir les états pelliculaires gras du cuir chevelu, ledit lysat correspondant à l'intégralité du lysat obtenu par lyse dudit microorganisme.

2. Utilisation selon la revendication 1 dans laquelle ledit lysat comprend de 0,1 à 50 % en poids en particulier de 1 à 20 % en poids de matière(s) active(s).

3. Utilisation selon l'une quelconque des revendications précédentes dans laquelle ledit lysat est administré par voie topique ou orale.

4. Utilisation selon l'une quelconque des revendications précédentes dans laquelle ledit lysat est en association avec au moins une quantité efficace d'au moins un microorganisme annexe, et/ou une de ses fractions, distinct dudit lysat.

5. Utilisation selon l'une quelconque des revendications précédentes dans laquelle le lysat est mis en oeuvre dans une composition se présentant sous forme d'une solution aqueuse, hydroalcoolique ou huileuse, d'une dispersion du type solution ou dispersion du type lotion ou sérum, d'une émulsion de consistance liquide ou semi-liquide du type lait, obtenue par dispersion d'une phase grasse dans une phase aqueuse (H/E) ou inversement (E/H), ou d'une suspension ou émulsion de consistance molle, semi-solide ou solide du type crème, de gel aqueux ou anhydre, ou encore de microémulsions, de microcapsules, de microparticules, ou de dispersions vésiculaires de type ionique et/ou non ionique.

6. Procédé cosmétique pour traiter et/ou prévenir les états pelliculaires gras du cuir chevelu chez un sujet comprenant au moins une étape d'administration audit sujet, d'un lysat d'au moins un microorganisme de l'espèce *Bifidobacterium longum,* ledit lysat correspondant à l'intégralité du lysat obtenu par lyse dudit microorganisme.

## Patentansprüche

1. Kosmetische Verwendung eines Lysats von mindestens einem Mikroorganismus der Gattung *Bifidobacterium longum* zur Behandlung und/oder Prävention der Zustände von Fettschuppen der Kopfhaut, wobei das Lysat der Gesamtheit des durch Lyse des Mikroorganismus erhaltenen Lysats entspricht.

2. Verwendung nach Anspruch 1, wobei das Lysat von 0,1 bis 50 Gew.-%, insbesondere von 1 bis 20 Gew.-% Wirksubstanze(en) umfasst.

3. Verwendung nach einem der vorangehenden Ansprüche, wobei das Lysat topisch oder oral verabreicht wird.

4. Verwendung nach einem der vorangehenden Ansprüche, wobei das Lysat in Kombination mit mindestens einer wirksamen Menge von mindestens einem Begleit-Mikroorganismus und/oder einer seiner Fraktionen, die von den Lysat verschieden ist, vorliegt.

5. Verwendung nach einem der vorangehenden Ansprüche, wobei das Lysat in einer Zusammensetzung eingesetzt wird, die in Form einer wässrigen, wässrig-alkoholischen oder öligen Lösung, einer Dispersion des Typs Lösung oder Dispersion des Typs Lotion oder Serum, einer Emulsion von flüssiger oder halbflüssiger Konsistenz, die durch Dispersion einer Fettphase in einer wässrigen Phase (O/W) oder umgekehrt (W/O) erhalten wird, oder einer Suspension oder Emulsion von geschmeidiger, halbfester oder fester Konsistenz vom Typ Creme, von wässrigem oder wasserfreiem Gel oder auch von Mikroemulsionen, Mikrokapseln, Mikroteilchen, oder vesikulären Dispersionen vom ionischen und/oder nichtionischen Typ vorliegt.

6. Kosmetisches Verfahren zur Behandlung und/oder Prävention der Zustände von Fettschuppen der Kopfhaut bei einer Testperson, umfassend mindestens einen Schritt der Verabreichung an die Testperson eines Lysats von mindestens einem Mikroorganismus der Gattung *Bifidobacterium longum,* wobei das Lysat der Gesamtheit des durch Lyse des Mikroorganismus erhaltenen Lysats entspricht.

## Claims

1. Cosmetic use of a lysate of at least one microorganism of the species *Bifidobacterium longum* to treat and/or prevent greasy dandruff conditions of the scalp, said lysate corresponding to the entirety of the lysate obtained by lysis of said microorganism.

2. The use according to claim 1, wherein said lysate comprises 0.1 to 50 % by weight, in particular 1 to 20 % by weight of active substance(s).

3. The use according to any of the preceding claims, wherein said lysate is administered via topical or oral route.

4. The use according to any of the preceding claims, wherein said lysate is in association with at least an efficient amount of at least one other microorganism, and/or one of the fractions thereof, differing from said lysate.

5. The use according to any of the preceding claims, wherein the lysate is used in a composition in the form of an aqueous, hydroalcoholic or oily solution, of a dispersion of solution type or dispersion of lotion or serum type, of an emulsion of liquid or semi-liquid consistency of milk type obtained by dispersion of a fat phase in an aqueous phase (O/W) or the reverse (W/O), or of a suspension or emulsion of soft, semi-solid or solid consistency of cream type or aqueous or anhydrous gel type, or further in the form of microemulsions, microcapsules, microparticles or vesicular dispersions of ionic and/or non-ionic type.

6. Cosmetic method for treating and/or preventing greasy dandruff conditions of the scalp in a subject, comprising at least a step to administer to said subject a lysate of at least one microorganism of the species *Bifidibacterium longum,* said lysate corresponding to the entirety of the lysate obtained by lysis of said microorganism.
